# EUROPEAN PATENT APPLICATION

(11) **EP 4 516 233 A1**
(43) Date of publication of application: **05.03.2025**
(21) Application number: 23193823.4
(22) Date of filing: 29.08.2023
(51) Int. Cl.: A61B 6/04, A61B 5/055, A61B 6/03, A61B 90/00

(54) **MEDICAL IMAGING SYSTEM**

(71) Applicant: Koninklijke Philips N.V., 5656 AG Eindhoven (NL)
(72) Inventor: SMINK, Jouke, Eindhoven (NL); KOKEN, Peter, Eindhoven (NL); STEHNING, Christian, 5656AG Eindhoven (NL); BECK, Gabriele, Eindhoven (NL)
(74) Representative: Philips Intellectual Property & Standards

(57) **Abstract**

The present invention relates to an imaging system (100) comprising a bore (110) for receiving an object (160) to be imaged, a table (120) for supporting the object (160) and configured to move longitudinally with respect to the bore (110), a position indicator (130) for indicating a position, and a controller (150). The controller (150) is configured to receive an image, acquired by the imaging system (100), of a region of interest (161) of the object (160) on the table (120). The image is acquired with the table (120) in a stored first table position (121) where the region of interest (161) of the object is at a reference position (111) inside the bore (110). The controller (150) is configured to receive user input marking an intervention location in the image, determine a puncture location (162), and calculate a second table position (122) wherein the puncture location (162) is marked with the position indicator. In this way, the efficiency of an image-guided workflow may be improved.

## Description

### FIELD OF THE INVENTION

The invention relates to an imaging system for medical imaging, in particular to an imaging system for an image-guided workflow. The invention further relates to a computer-implemented method for controlling a table with respect to an imaging system. The invention also relates to a computer program element and to a computer readable medium having stored thereon the computer program element.

### BACKGROUND OF THE INVENTION

Large imaging systems such as Magnetic Resonance Imaging (MRI) or X-ray Computed Tomography (CT) may provide superb clinical imaging for diagnosis and therapy. However, access to the patient is a potential drawback of image-guided interventions utilizing such imaging systems because the area of interest is typically placed in the center of a long and narrow bore during imaging. This may be of particular importance for needle interventions, such as for biopsies or localized treatment, where it may be a challenge to reach inside the imaging system bore. As an example, a surgeon may have to bow into the bore to with (real-time) imaging check a location for incision. This may be done by pushing the finger into the potential incision region. After needle placement this location may not be accurate enough and a replacement of the needle may be required.

Image-guided procedures on patients being imaged with an imaging system having a bore, such as MRI or CT, may therefore be inefficient and result in unsatisfactory interventionalist ergonomics with uncomfortable and/or impractical working positions. Furthermore, other aspects such as patient throughput, and ultimately medical outcomes may also be sub-optimal.

Hence, there is a need to improve the workflows of such image guided interventions.

### SUMMARY OF THE INVENTION

It is an object of the invention to provide an improved image guided workflow.

The invention is defined by the independent claims. Advantageous embodiments are defined in the dependent claims.

According to a first aspect, there is provided an imaging system for medical imaging. The imaging system comprises: a bore for receiving an object to be imaged; a table for supporting the object and configured to move longitudinally with respect to the bore; a position indicator for indicating a position; and a controller. The controller may e.g. comprise a memory and a processor. The controller is configured to:
- receive an image, acquired by the imaging system, of a region of interest of the object on the table, wherein the table is positioned in a stored first table position, wherein in the first table position the region of interest of the object is at a reference position inside the bore;
- receive user input marking an intervention location in the image;
- determine a puncture location on the object relative to the reference position; and
- calculate and store a second table position, based on the puncture location, the position indicated by the position indicator, and the stored first table position, such that when the table is in the second table position, the puncture location on the object coincides with the position indicated with the position indicator.

By determining a puncture (or incision or similar) location on the object (such as a patient) based on user input to the image and subsequently calculating a second table position where that location is marked with the position indicator, the efficiency of a workflow may be improved. The second table position can be automatically determined. When the table is moved to the second table position, the puncture location is indicated with the position indicator. Preferably the table may be automatically moved by the controller, but other options are also possible. Such as manual or semi-automatic movement of the table to a determined coordinate. The second position is preferably such that the puncture location of the object on the table in the second position is outside of the bore of the imaging system. In this way, a user does not have to stretch into the bore to see the indication of the puncture location.

The reference position inside the bore may be at an isocenter of the bore or close to an isocenter of the bore for optimal image acquisition of the region of interest.

Determining a puncture location relative to the reference position may comprise receiving user input to mark the puncture location. Such as e.g. receiving user input to the image with the marked intervention location. As an example, the user may indicate a suitable path between the intervention location and a puncture location. Alternatively, or additionally, the controller may translate the marked intervention location in the image to a puncture location on the object relative to the reference position via computational road-mapping of the internal volume of the object on the table. The translation may comprise to calculate, based on an imaged volume of the object, an optimal path for an intervention needle, catheter or similar instrument from the puncture location on the object to the intervention location inside the object. Translating the intervention location in the image to determine a puncture location on the object may be based on anatomical regions that can be passed or needs to be avoided by e.g. a catheter. Determination of a puncture location may include the use of artificial intelligence algorithms, such that algorithms that can learn from previous intervention procedures and/or strategies. The controller may be configured to automatically determine the puncture location or provide a recommendation for a user providing input.

Hence, machine learning or artificial intelligence may be integrated for providing and suggesting an optimal puncture location and trajectory to the surgeon. Data mining taking a large set of image guided interventions into account can be used to determine and integrate puncture locations and catheter trajectories used in clinical practice into an artificial intelligence network, such as e.g. a deep learning network. Personalized preferences including avoidance of anatomical and physiological areas may be taken into account.

The position indicator may be a stationary indicator, such as but not limited to a laser bow or bridge, e.g. positioned close to the bore of the imaging system. Other stationary solutions, such as position indicators attached to the bore of the imaging system or to a wall, a floor, or a roof of the imaging room etc. are also possible. The laser bridge may be a standard laser bridge, commonly used for patient positioning e.g. for therapy or therapy planning. Alternatively, the position indicator may be mobile. Such as but not limited to a position indicator attached to a mobile unit like a robotic device. E.g. a robotic device for performing interventions. An intervention robotic device may be positioned on a rail mounted on the table, which allows movement of the intervention robotic device to the incision and treatment region. An improved positioning of the intervention robotic device may be accomplished with the position indicated by the position indicator overlapping with a translated incision location of the robotic device. The position indicator may use a laser or other light sources to indicate e.g. a spot or a line on the object on the table.

The controller may e.g. comprise a processor and a memory. The processor may be a single and/or multi core processing unit, a graphics processing unit, an accelerated processing unit, a digital signal processor, a field programmable gate array, and/or an application-specific integrated circuit, etc. Processing may be carried out involving a single apparatus, such as e.g. a single controller comprising a processor, or may be carried out by a distributed system with multiple local and/or remote units. The memory may be a short-term and/or a long-term memory configured to interact with the processor.

According to an embodiment of the invention, the controller is configured to calculate and store an updated coordinate for the position indicator, such that when the table is in the second table position and the position indicator indicates an updated position with the updated coordinate, the puncture location on the object coincides with the updated position indicated with the position indicator. This is advantageous in cases the position that is indicated with the position indicator can be updated, such as by moving a mobile position indicator and/or changing the target spot or line that the indicator marks. Such as by pivoting an indicator mounted on e.g. a wall or roof. When the position indicated by the position indicator can move, it may be possible to reduce the absolute movement of the patient table, speed up the workflow and provide additional flexibility. As an example, a mobile position indicator such as a robotic device, may move into the bore of the imaging system. E.g. the system may have rails on which the position indicator can move with respect to the bore and/or the table. When the position indicator on a robotic surgical device may move into the bore, the second table position may advantageously be similar or the same to the first table position such that table movement is reduced.

According to an embodiment of the invention, the controller is configured to store a temporary table position, wherein when the table is in the temporary table position the region of interest coincides with the position indicated with the position indicator, and wherein the controller is configured to calculate and store the first table position based on the temporary table position and an offset between the position indicated with the position indicator and the reference position. In this way, the system may automatically determine the first table position. An operator may move the table to the temporary table position such that the region of interest that the operator wants to image is indicated with the position indicator. This may be very intuitive for the operator since the region is directly indicated e.g. under a laser bridge. Once the table is in the correct temporary position, that position may be stored, and the first table position may be automatically determined. The first table position may then subsequently be used when the region of interest is imaged, since in the first table position the region of interest of the object is at a reference position inside the bore. This helps to provide for an efficient and intuitive workflow.

According to an embodiment of the invention, the controller is configured to store a third table position, wherein when the table is in the third table position the table is further outside of the bore compared to in the first position, and wherein the region of interest of the object on the table in the third table position is not at an isocenter of the bore. It may be advantageous to determine a third table position where e.g. an interventionalist may ergonomically have access to the object on the table, since the table is further outside the bore. During an image guided procedure, it may be advantageous to toggle between the third position for good access to the object (patient) and the first position for optimal imaging of the object (patient).

According to an embodiment of the invention, the imaging system is a magnetic resonance imaging system. The invention may be particularly advantageous for magnetic resonance imaging systems, since such systems often have long and narrow bores that are hard to reach into.

According to an embodiment of the invention, the controller is configured to acquire an off-center image of the object, acquired by the imaging system, when the table is in the third table position. The controller is configured to geometrically correct the off-center image. The third table position is further outside the bore compared to the first table position. The region of interest of the object on the table is not at an isocenter of the bore when the table is in the third position. Access to the object may be improved in the third table position. However, it may be advantageous if the object can also be imaged when on the table in the third table position. Off-center imaging may lead to distortions and signal voids of the region of interest. By receiving an off-center image of the object, i.e. an image wherein the imaged part of the object is not at an isocenter of the bore, and geometrically correct said image, it may be possible to image the procedure with geometrically correct images also when the table is in the ergonomically advantageous third position. Correction of geometric differences when the table is in the third table position may be fine-tuned based on differences of acquired images in the third table position as compared to images acquired in the first table position.

According to an embodiment of the invention, the imaging system is a magnetic resonance imaging system and the geometric correction of the off-center image comprises correcting for gradient non-linearities of the magnetic resonance system and/or comprises comparing the off-center image with an image acquired with the table at the first table position. Based on e.g. system characteristics, gradient non-linearity etc., it may be possible to calculate and set e.g. B0 shim, limiting image off-center values etc.

According to an embodiment of the invention, the imaging system comprises a display, and the controller is configured to provide the image on the display. This embodiment is advantageous since the system may directly present the image of the region of interest on the display, such that a user may provide input. The display may be positioned e.g. on the bore or in proximity to the bore of the imaging system such that it may be viewed when standing next to the imaging system. Alternatively, or additionally the system may comprise a display located remotely, e.g in another room or at another site.

According to an embodiment of the invention, the imaging system comprises a user interface. The user interface may include e.g. a speech interface, a gesture recognition interface, a touch interface, a mouse, a hand switch, or a foot switch. The user interface may advantageously be used to provide input to e.g. the table position, the intervention location, selection of imaging parameters etc. In case the system comprises a display, a user may interact with e.g. an image on the display via the user interface.

According to an embodiment of the invention, the imaging system comprises a camera directed towards the table, and the controller is configured to update a stored table position and/or a stored position of an object on the table based on an image from the camera. The camera may be e.g. a 2D or 3D camera, a camera registering visible and/or infrared radiation etc. The camera may advantageously improve the efficiency and accuracy of the workflow by determining the position of the object on the table with respect to the table, the table position e.g. with respect to the bore etc. As one example, the camera may provide input with respect to a change of position e.g. in case the object moves on the table. In this way, the accuracy of the workflow may be improved.

According to a second aspect of the invention, there is provided a computer implemented method for controlling a table with respect to an imaging system comprising a bore and a position indicator, the method comprising:
- receiving an image, acquired with the imaging system, of a region of interest of an object on the table, wherein the image is acquired with the table positioned in a stored first table position, wherein in the first table position the region of interest of the object is at a stored reference position inside the bore;
- receiving user input marking an intervention location in the image;
- determining a puncture location on the object relative to the reference position; and
- determining a second table position based on the puncture location, a stored position indicated by the position indicator, and the first table position, such that when the table is in the second table position the puncture location on the object coincides with the position indicated with the position indicator.

According to an embodiment of the invention, the method further comprises:
- storing a temporary table position, wherein in the temporary table position the region of interest on the object coincides with the position indicated with the position indicator, and
- determining the first table position based on the temporary table position and an offset between the position indicated with the position indicator and the reference position inside the bore.

According to an embodiment of the invention, the step of determining a puncture location on the object relative to the reference position comprises road-mapping of an internal volume of the object and/or determining the puncture location (from the intervention location) using a trained artificial intelligence algorithm. Determination of the puncture location, such as via a translation from an intervention location in the image to a puncture location, may comprise to calculate, based on an imaged volume of the object, an optimal path for an intervention needle, catheter or similar instrument from the puncture location on the object to the intervention location inside the object. Translating the intervention location in the image to a puncture location on the object may be based on anatomical regions that can be passed or needs to be avoided by e.g. a catheter. Determination of a puncture location may include the use of artificial intelligence algorithms, such that algorithms that can learn from previous intervention procedures and/or strategies. Computational road-mapping may e.g. automatically determine the puncture location or provide recommendations as to suitable options.

According to a third aspect of the invention, there is provided a computer program element, which, when being executed by a controller, is adapted to cause the controller to perform the method as described above.

According to a fourth aspect of the invention, there is provided a computer-readable medium having stored thereon the computer program element.

These and other aspects of the invention will be apparent from and elucidated with reference to the embodiments described hereinafter.

### BRIEF DESCRIPTION OF THE DRAWINGS

Fig. 1 schematically illustrates an imaging system.
Fig. 2 schematically illustrates a workflow with an imaging system.
Fig. 3 schematically illustrates a workflow with an imaging system.
Fig. 4 schematically illustrates needle positioning with respect to a lesion.
Fig. 5 shows a flow diagram of a computer implemented method.
Fig. 6 shows a flow diagram of a computer implemented method.

### DETAILED DESCRIPTION OF EMBODIMENTS

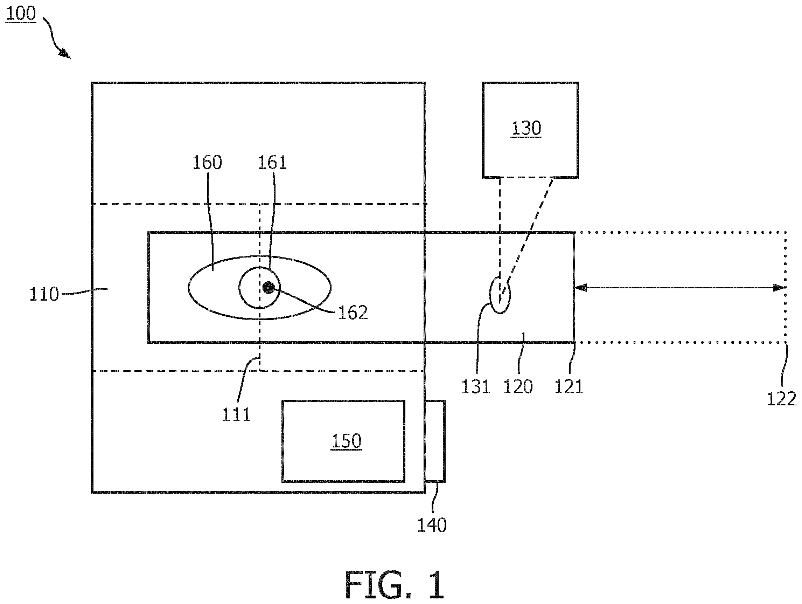

An imaging system 100, according to embodiments of the invention, is schematically illustrated in Fig. 1. The imaging system 100 includes a bore 110, which may be relatively long and narrow such that it can fit a patient or the parts of the patient to be imaged. An example of a type of imaging systems where a long and narrow bore so commonly used in order to achieve good imaging quality is magnetic resonance imaging systems. However, also other tomographic imaging systems, such as computed tomography systems, positron emission tomography systems, single-photon emission computed tomography systems, combinations of such systems etc. may also have long and narrow bores. In the bore 110 of the imaging system 100, a reference position 111 is defined. The reference position may be e.g. at an isocenter of the bore or close to the center of the bore.

The imaging system 100 includes a table 120. The table may move longitudinally in and out of the bore or closer to or further away from the bore. An object 160 to be imaged, such as a patient to be imaged, may be positioned on the table 120. As illustrated in Fig.1, the table is configured to move between at least a first table position 121 and a second table position 122. The table positions are defined, e.g. with one or more coordinates in relation to the reference position 111 in the bore.

The imaging system includes a position indicator 130 that is configured to indicate a position 131. The indicated position 131, such as the center point or center line of the indicated position 131, may also be defined in the same coordinate system as the table positions and the reference position 111. The indicated position may e.g. take the form of a line or a dot or a circle or an oval etc. In the schematic drawing in Fig. 1, the indicated position 131 is visible on the table 120. However, if an object 160 on the table is located at the position 131, the position indicator will mark a location 131 on the object. The indicated position 131 may be fixed in the coordinate system, such as if the position is indicated with a laser bow that is fixed with respect to the imaging system. Alternatively, the indicated position 131 may be altered by the position indicator 130, such that positions at various coordinates may be indicated. The coordinate system for the positions with respect to imaging system 100 may be a one-dimensional coordinate system along the longitudinal axis of the table 120. Other coordinate systems are also possible, such as e.g. a two-dimensional system in a plane of the table 120.

The imaging system includes a controller 150. The controller may e.g. include an ASIC, a FPGA, and/or a processor and a memory storing instructions for controlling the processor. In the example in Fig. 1, the imaging system includes a local display 140. The imaging system may in some examples also include a user interface (not shown in Fig. 1). The user interface may include e.g. a speech interface, a gesture recognition interface, a touch interface, a mouse, a hand switch, or a foot switch. Such a user interface may be used to provide user input to e.g. the table position, the intervention location, selection of imaging parameters etc. In case the system comprises a display, like in Fig. 1, and a user interface, a user may interact with e.g. an image on the display via the user interface.

Fig. 1 illustrates an object 160, such as a patient, on table 120 in the bore 110. The object 160 has a region of interest 161 that may be imaged by the imaging system 100. Based on imaging of the region of interest 161 and user input marking an intervention location in at least one image, the controller 150 may determine a puncture location 162 in the coordinate system that is relative to the reference position 111. When the puncture location 162 is known, the second table position 122 may be determined such that when the table 120 is in the second table position 122, the puncture location 162 is at the position 131 indicated by the position indicator 130.

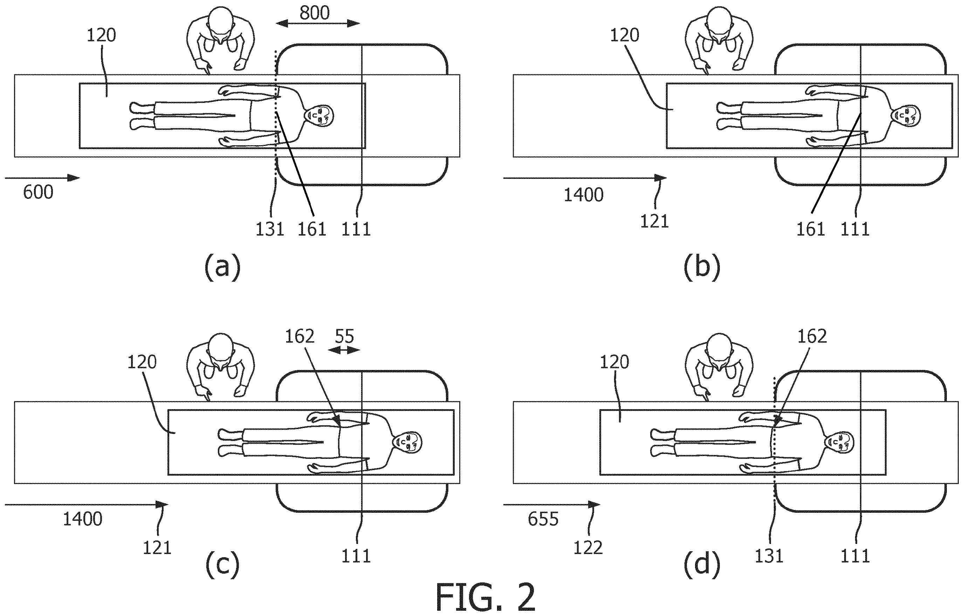

Fig. 2 shows an example of a workflow with the imaging system 100. The positions in Fig. 2 are illustrated with one-dimensional coordinates from the leftmost side of the imaging system 100 and from the reference position 111. In this case, the position indicator 130 is a stationary light visor with a fixed indicated position 131.

In Fig. 2, at (a), the table has been moved to a table position with coordinate 600, where the region of interest 161 of the patient is below the position 131 indicated by the light visor. Based on the table coordinate and the distance between the reference position 111 and the indicated position 131, shown as 800 in Fig. 2, at (a), a first table position 121 may be determined and stored. In this case the first table position 121 has coordinate 1400.

Consequently, as shown in Fig. 2, at (b), when the table with the patient has been moved to the first table position 121 (with coordinate 1400), the region of interest 161 of the patient is positioned at the reference position 111. The patient has moved further into the bore 110. In this example, the reference position is at the isocenter of the bore 110. With the region of interest 161 at the isocenter of the bore 110, the patient may be imaged by the imaging system 100 to generate one or multiple images of the region of interest.

A user, such as an interventionalist, may review and interact with the generated images in viewing software. Based on input from the user an entry point or puncture location 162 may be determined, as illustrated in Fig. 2 at (c). With the determined puncture location 162 in relation to the reference position 111, here shown as a difference of 55 distance units, and the known coordinate of the light visor in relation to the reference position (here -800), a second table position 122 may be determined (at 655) and stored.

Fig. 2, at (d) shows the table in the second table position 122. Now the puncture location 162 is directly underneath the position 131 indicated by the light visor, which may assist the interventionalist in quickly finding the right location and performing an intervention or part of an intervention, such as placing a needle at the puncture location 162. For simplicity, the location 131 is here indicated with a line. However, e.g. indication of a spot, such as in combination with a two-dimensional coordinate system is also possible.

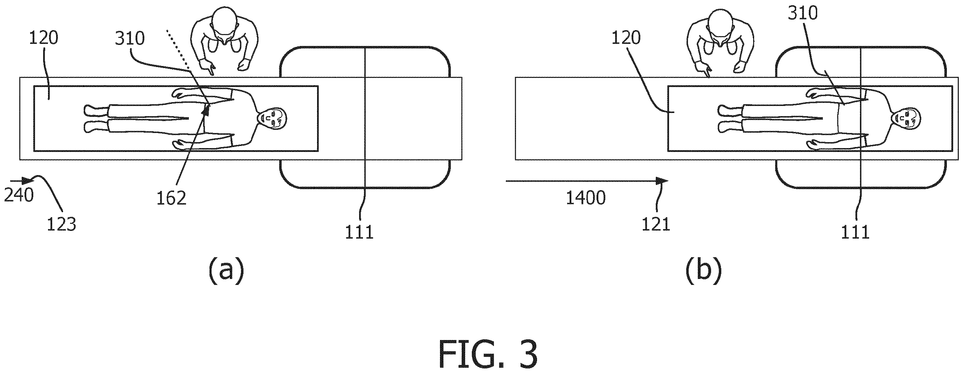

Fig. 3 illustrates additional steps of a workflow, such as the workflow shown in Fig. 2. In Fig. 3, at (a), the table with the patient has been moved further out of the bore 110 to a third table position 123. The system may store the coordinate (here 240) of the third table position in the memory. This third table position 123 is a position which may comfortable and ergonomic for the interventionalist to perform an intervention without having to stretch into the bore 110. In the schematic of Fig. 3, the interventionalist or other user is placing a needle 310 at the puncture location 162.

In Fig. 3, at (b), the table has been moved back to the first table position 121. In this position the region of interest 161, here with a needle 310, may be imaged with the imaging system 100 in order to check or confirm progress of the intervention. If there is a display on or close by the imaging system 100, the interventionalist may be able to view the images without having to move to a different location. As both the first table position 121 and the second table position 123 are stored, the user of the imaging system 100 may easily toggle the table between a comfortable working position and an imaging position. The table may be automatically moved between the positions, such as at the command of the user via a user interface.

As an example, an operator of e.g. a magnetic resonance imaging (MRI) system with a light visor, may define with the system one or more of the following table positions:
1. The area of interest indicated by the light visor in the isocenter or close to the isocenter.
2. The entry point for a needle as indicated in an MRI scan, is underneath the laser (such as a standard laser of the scanner or a laser bridge in an MRI-radiation therapy setup)
3. The entry point of the needle outside the magnet at a comfortable working location for the interventional operator
4. The entry point of a catheter at a location on the patient that is not covered by the roadmap scan that depicts the lesion, is at the isocenter or close to the isocenter

These positions may be indicated on a touch screen at the magnet and the operator can use e.g. buttons, gesture control or voice control to instruct the table to travel to the desired location.

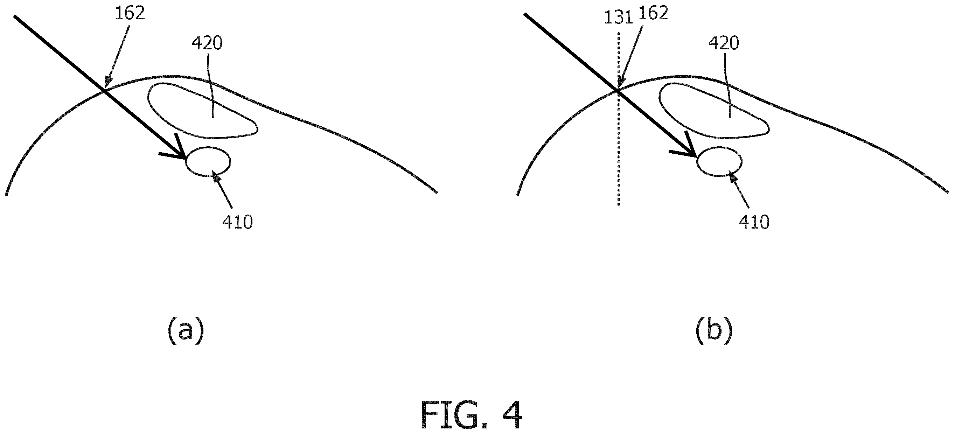

Fig. 4 schematically shows an example of a part of a patient anatomy with a needle entry position or puncture location 162, in relation to a lesion 410 to be treated. The interventional radiologist may want to puncture a lesion in an organ such as for instance the liver or kidney. The patient may be moved into the bore of the imaging system (table in first position 121) and a roadmap scan is made of the region of interest, where the roadmap scan shows the location of the lesion 410. Using one or multiple images from the scan, a plan can be made of what the best location is to enter the body with a needle, catheter or similar. The location of the lesion 410 is translated to a puncture location 162. To avoid sensitive areas 420, this may not be the straight, shortest trajectory but e.g. under a certain angle, and/or with a curved trajectory etc. Translation from the intervention location to a puncture location 162, taking anatomical information to pass or to avoid passing areas of the patient anatomy, may leverage artificial intelligence. In a second step, as illustrated in Fig. 4 (b), the table may be moved to a second table position 122, such that entry point 162 is being marked with the position location 131 on the skin.

As a further step, not shown in Fig. 4, the patient may be moved out of the bore a bit further (e.g. to third table position 123) to conveniently prepare the skin, apply local anesthesia, create a small incision and place the needle. Once the needle is inserted for a few mm, the patient may be moved inside the scanner again (first table position 121) and with imaging the correct placement and direction can be confirmed. The patient may be moved out again to the work position (third table position 123) to advance the needle in the right direction based on the information taken from the imaging scans. Such a process of toggling between imaging position and working position may be repeated until the needle has reached the target as confirmed by imaging system.

Several variations to the workflows with the imaging system are conceivable. E.g. the table may be positioned in an ergonomic image guidance position that is at an image off center position where overview images may be provided. The overview images at image guidance position may be geometrically corrected, e.g. by taking other overview images into account. In the case of MRI, the geometric correction may take gradient magnet non-linearities into account.

A surgeon's commands to e.g. scan, reposition table etc. may be determined by the system using a bore microphone, camera, (foot) switch, touch screen or similar. The surgeon's commands may be used to guide catheter placement, e.g. via catheter guidance robotics using such user interfaces.

The catheter position may be tracked and visualized on an in/on bore screen. The surgeon may initiate scanning, table repositioning between the initial catheter placing position, an ergonomic image off-center position or an isocenter position by e.g. vocal commands or gestures. Such as with artificial intelligence-based speech and/or gesture recognition.

Any part of the workflow may include the use of catheter guidance robotics, e.g. instead of manual catheter placement.

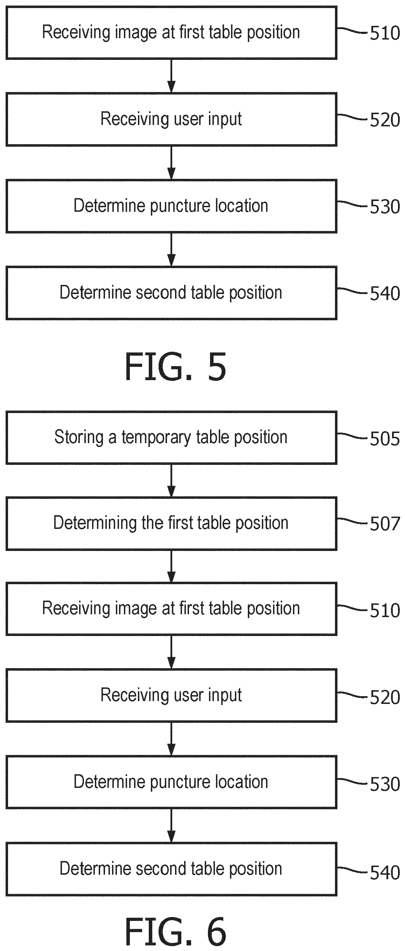

Fig. 5 illustrates an example of a computer-implemented method with a flow chart. The computer-implemented method is suitable for controlling a table 120 with respect to an imaging system 100 comprising a bore 110 and a position indicator 130. The method includes:
- receiving 510 an image, acquired with the imaging system 100, of a region of interest 161 of an object 160 on the table 120, wherein the image is acquired with the table 120 positioned in a stored first table position 121, wherein in the first table position 121 the region of interest 161 of the object 160 is at a stored reference position 111 inside the bore 110;
- receiving 520 user input marking an intervention location in the image;
- determining 530 a puncture location 162 on the object relative to the reference position 111; and
- determining 540 a second table position 122 based on the puncture location 162, a stored position 131 indicated by the position indicator 130, and the first table position 121, such that when the table 120 is in the second table position 122 the puncture location 162 on the object coincides with the position indicated 131 with the position indicator 130.

Fig. 6 shows an example of a method that is similar to the method in Fig. 5. However, in this example the method includes two additional steps. The method includes:
- storing 505 a temporary table position, wherein in the temporary table position the region of interest 161 on the object 160 coincides with the position indicated 131 with the position indicator 130;
- determining 507 the first table position 121 based on the temporary table position and an offset between the position indicated 131 with the position indicator 130 and the reference position 111 inside the bore 110;
- receiving 510 an image, acquired with the imaging system 100, of a region of interest 161 of an object 160 on the table 120, wherein the image is acquired with the table 120 positioned in a stored first table position 121, wherein in the first table position 121 the region of interest 161 of the object 160 is at a stored reference position 111 inside the bore 110;
- receiving 520 user input marking an intervention location in the image;
- determining 530 a puncture location 162 on the object relative to the reference position 111; and
- determining 540 a second table position 122 based on the puncture location 162, a stored position 131 indicated by the position indicator 130, and the first table position 121, such that when the table 120 is in the second table position 122, the puncture location 162 on the object coincides with the position indicated 131 with the position indicator 130.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

## Claims

1. Imaging system (100) for medical imaging, the imaging system comprising:
a bore (110) for receiving an object (160) to be imaged;
a table (120) for supporting the object (160) and configured to move longitudinally with respect to the bore (110);
a position indicator (130) for indicating a position (131); and
a controller (150) configured to:
- receive (510) an image, acquired with the imaging system (100), of a region of interest (161) of the object (160) on the table (120), wherein the image is acquired with the table positioned in a stored first table position (121), wherein in the first table position (121) the region of interest (161) of the object (160) is at a reference position (111) inside the bore;
- receive (520) user input marking an intervention location in the image;
- determine (530) a puncture location (162) on the object relative to the reference position (111); and
- calculate and store (540) a second table position (122), based on the puncture location (162), the position (131) indicated by the position indicator, and the stored first table position (121), such that when the table is in the second table position (122), the puncture location (162) on the object coincides with the position (131) indicated with the position indicator (130).

2. The imaging system according to claim 1, wherein the controller (150) is configured to calculate and store an updated coordinate for the position indicator (130), such that when the table is in the second table position (122) and the position indicator (130) indicates an updated position (131) with the updated coordinate, the puncture location (162) on the object coincides with the updated position (131) indicated with the position indicator (130).

3. The imaging system according to claim 1 or 2, wherein the controller (150) is configured to store (505) a temporary table position, wherein when the table is in the temporary table position the region of interest (161) coincides with the position (131) indicated with the position indicator (130), and
wherein the controller (150) is configured to calculate and store (507) the first table position (121) based on the temporary table position and an offset between the position (131) indicated with the position indicator (130) and the reference position (111).

4. The imaging system according to claim 1 or 2 or 3, wherein the controller (150) is configured to store a third table position (123), wherein when the table is in the third table position (123) the table is further outside of the bore (110) compared to in the first position (121), and wherein the region of interest (161) of the object on the table in the third table position (123) is not at an isocenter of the bore.

5. The imaging system according to any of the preceding claims, wherein the imaging system (100) is a magnetic resonance imaging system.

6. The imaging system according to claim 4, wherein the controller (150) is configured to receive an off-center image of the object (160), acquired by the imaging system when the table (120) is in the third table position (123), and wherein the controller (150) is configured to geometrically correct the off-center image.

7. The imaging system according to claim 6, wherein the geometric correction of the off-center image comprises correcting for gradient non-linearities of a magnetic resonance system and/or the geometric correction comprises comparing the off-center image with an image acquired with the table at the first table position (121).

8. The imaging system according to any of the preceding claims, wherein the imaging system comprises a display (140), and wherein the controller is configured to provide the image on the display (140).

9. The imaging system according to any of the preceding claims, wherein the imaging system comprises a user interface.

10. The imaging system according to any of the preceding claims, wherein the imaging system comprises a camera directed towards the table (120), and wherein the controller is (150) configured to update a stored table position and/or a stored position of an object (160) on the table based on an image from the camera.

11. A computer-implemented method for controlling a table (120) with respect to an imaging system (100) comprising a bore (110) and a position indicator (130), the method comprising:
- receiving (510) an image, acquired with the imaging system, of a region of interest of an object on the table, wherein the image is acquired with the table positioned in a stored first table position, wherein in the first table position the region of interest of the object is at a reference position inside the bore;
- receiving (520) user input marking an intervention location in the image;
- determining (530) a puncture location on the object relative to the reference position; and
- determining (540) a second table position based on the puncture location, a stored position indicated by the position indicator, and the first table position, such that when the table is in the second table position, the puncture location on the object coincides with the position indicated with the position indicator.

12. The method of claim 11, wherein the method further comprises:
- storing (505) a temporary table position, wherein in the temporary table position the region of interest on the object coincides with the position indicated with the position indicator, and
- determining (507) the first table position based on the temporary table position and an offset between the position indicated with the position indicator and the reference position inside the bore.

13. The method of claim 11 or 12, wherein determining (530) the puncture location comprises road-mapping of an internal volume of the object and/or determining the puncture location using a trained artificial intelligence algorithm.

14. A computer program element, which, when being executed by a controller, is adapted to cause the controller to perform the method according to claim 11 or 12 or 13.

15. A computer-readable medium having stored thereon the computer program element of claim 14.
